# EUROPEAN PATENT SPECIFICATION

(11) **EP 4 519 236 B1**
(45) Date of publication and mention of the grant of the patent: **01.07.2026**
(21) Application number: 23725152.5
(22) Date of filing: 04.05.2023
(51) Int. Cl.: C07C 45/63, C07C 49/333

(54) **CHEMICAL PROCESS**
CHEMISCHES VERFAHREN
PROCÉDÉ CHIMIQUE

(30) Priority: 05.05.2022 GB 202206545
(43) Date of publication of application: 12.03.2025
(73) Proprietor: Syngenta Crop Protection AG, 4058 Basel (CH)
(72) Inventor: KREITUSS, Imants, 4333 Münchwilen (CH)
(74) Representative: SYNGENTA IP
(86) International application number: PCT/EP2023/061760
(87) International publication number: WO 2023/213926

(56) References cited:
- DALTON ET AL.: "Bromohydrin Formation in Dimethyl Sulfoxide. V. The Reaction of Norbornene", J. ORG. CHEM, vol. 37, no. 3, 1972, pages 362 - 7, XP093073783

## Description

The present invention relates to a novel process for the synthesis of certain α-halo norcamphor compounds as well as novel processes to make norcamphor. Such compounds are useful intermediates in the synthesis of herbicidal propynyl-phenyl compounds, which are known, for example from WO 2015/197468 and processes for making such compounds or intermediates thereof are also known.

The halogenation of norcamphor or derivatives thereof is known, see for example WOODS and ROBERTS, Bromination Rates of Some Norcamphor Derivatives, J. Org. Chem 1957, Vol. 22, p. 1124-6, MCDONALD and TABOR, Molecular Rearrangements, J. Org. Chem 1968, Vol. 33(7), p. 2934-41 and TOBLER et al., The Reaction of Norbornene with t-Butyl Hypochlorite, J. Org. Chem 1964, Vol. 29(10), p. 2834-8.

Typically the halogenation of norcamphor is carried out in acetic acid, see for example PEREZ et al. Molybdenum(0)-Catalyzed Reductive Dehalogenation of α-Halo Ketones with Phenylsilane, J. Org. Chem 1987, Vol. 52(25), p. 5570-4 or DALTON et al., Bromohydrin Formation in Dimethyl Sulfoxide. V. The Reaction of Norbornene, J. Org. Chem 1972, Vol. 37(3), p. 362-7, however this often results in the need for further purification of the product due to overbromination and poor selectivity. Alternative solvents for the halogenation of norcamphor are also known, see for example GAUZE et al, Proton Chemical Shifts in Some gem-Difunctional Compounds: 3-endo- and 3-exo-Substituted Norbornanones, J. Phys. Org. Chem. 2006, Vol. 19, p. 376-383 or KOVAL'SKAYA et al. Zhurnal Obshchei Khimii 1992, Vol. 62(4), p. 878-84 but these are either unsuitable for large scale production and/or have a high environmental footprint due to yield losses and the need for additional purification of the product. Thus, such approaches are not ideal for large scale production and therefore a new, more efficient synthesis method is desired to avoid the generation of undesirable by-products in a more environmentally sustainable way.

The present invention provides a process for the selective halogenation of norcamphor which (i) significantly limits overhalogenation and (ii) reduces the need to use an excess of halogenating reagent to achieve full conversion. Surprisingly, we have now found that a selective halogenation to deliver the desired mono halogenated product, a compound of formula (I), can be achieved in the process of the present invention which in turn can be converted to the desired propynyl-phenyl herbicidal compounds. Such a process also allows for process telescoping, which may be more cost effective, environmentally friendly and produce less waste products.

Thus, according to the present invention there is provided a process for the preparation of a compound of formula (I), 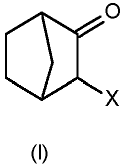 wherein X is halogen;
said process comprising:
reacting a compound of formula (II), 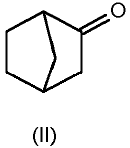
with a halogenating reagent in a suitable reaction medium comprising an organic carbonate, to give a compound of formula (I).

As used herein, the term "halogen" refers to fluorine (fluoro), chlorine (chloro), bromine (bromo) or iodine (iodo).

As used herein, the term "hydroxyl" or "hydroxy" means an -OH group.

As used herein, the term "C₁-C₆alkyl" refers to a straight or branched hydrocarbon chain radical consisting solely of carbon and hydrogen atoms, containing no unsaturation, having from one to six carbon atoms, and which is attached to the rest of the molecule by a single bond. C₁-C₄alkyl and C₁-C₂alkyl are to be construed accordingly. Examples of C₁-C₆alkyl include, but are not limited to, methyl, ethyl, *n*-propyl, 1-methylethyl (iso-propyl), *n*-butyl, and 1-dimethylethyl (*t*-butyl).

A "C₁-C₂alkylene" group refers to the corresponding definition of C₁-C₂alkyl, except that such radical is attached to the rest of the molecule by two single bonds. Examples of C₁-C₂alkylene, are -CH₂- and - CH₂CH₂-.

As used herein, the term "C₁-C₆alkoxy" refers to a radical of the formula -ORₐ where Rₐ is a C₁-C₆alkyl radical as generally defined above. C₁-C₄alkoxy is to be construed accordingly. Examples of C₁₋₄alkoxy include, but are not limited to, methoxy, ethoxy, propoxy, iso-propoxy and *t*-butoxy.

As used herein, the term "C₂-C₆alkenyl" refers to a straight or branched hydrocarbon chain radical group consisting solely of carbon and hydrogen atoms, containing at least one double bond that can be of either the (*E*)- or (*Z*)-configuration, having from two to six carbon atoms, which is attached to the rest of the molecule by a single bond. C₂-C₄alkenyl is to be construed accordingly. Examples of C₂-C₆alkenyl include, but are not limited to, prop-1-enyl, allyl (prop-2-enyl) and but-1-enyl.

As used herein, the term "C₂-C₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. Examples of C₃-C₆alkynyl include, but are not limited to, prop-1-ynyl and propargyl (prop-2-ynyl).

As used herein, the term "C₁-C₃alkoxyC₁-C₃alkyl-" refers to a radical of the formula R_{b}-O-Rₐ- where R_{b} is a C₁-C₃alkyl radical as generally defined above, and Rₐ is a C₁-C₃alkylene radical as generally defined above.

As used herein, the term "halogenating reagent" refers to any chemical reagent capable of introducing a halogen atom in the target molecule by forming a carbon-halogen bond.

As used herein, the term "reaction medium" refers to any solvent or mixture of solvents that are inert under the reaction conditions. The skilled person would appreciate that reactants and reagents used in the processes of the invention may also act as a solvent.

As used herein, the term "organic carbonate" refers to any organic reaction medium comprising a carbonate ester functional group -O-C(=O)-O- and may be linear or cyclic. Examples of organic carbonates include, but are not limited to, dimethyl carbonate (DMC), diethyl carbonate (DEC), ethylene carbonate (EC), propylene carbonate (PC), butylene carbonate (BC) and glycerol carbonate (GyC).

The process of the present invention can be carried out in separate process steps, wherein the intermediate compounds can be isolated at each stage. Alternatively, the process can be carried out in a one-step procedure wherein the intermediate compounds produced are not isolated. Thus, it is possible for the process of the present invention to be conducted in a batch wise or continuous fashion.

The following list provides definitions, including preferred definitions, for substituents X, X^{a}, G and R¹ with reference to the process according to the invention. For any one of these substituents, any of the definitions given below may be combined with any definition of any other substituent given below or elsewhere in this document.

X is halogen. Preferably, X is chlorine, bromine or iodine. More preferably, X is chlorine or bromine. Most preferably X is bromine.

G is selected from the group consisting of hydrogen, C₂-C₆alkenyl, C₂-C₆alkynyl, C₁-C₃alkoxyC₁-C₃alkyl-, -C(O)-R¹, -C(O)-X^{a}-R¹ and -S(O)₂-R¹. Preferably, G is selected from the group consisting of hydrogen, -C(O)-R¹, -C(O)-X^{a}-R¹ and -S(O)₂-R¹. More preferably, G is selected from the group consisting of hydrogen, -C(O)-R¹ and -C(O)-X^{a}-R¹. Even more preferably, G is hydrogen or -C(O)-R¹. Most preferably, G is -C(O)-R¹.

X^{a} is oxygen or sulfur. Preferably, X^{a} is oxygen.

R¹ is selected from the group consisting of C₁-C₆alkyl, C₂-C₆alkenyl, phenyl and 4-fluorophenyl. Preferably, R¹ is selected from the group consisting of C₁-C₆alkyl, C₂-C₆alkenyl and phenyl. More preferably, R¹ is selected from the group consisting of C₁-C₆alkyl and C₂-C₆alkenyl. Even more preferably, R¹ is C₁-C₆alkyl.

Scheme 1 below describes the reactions of the invention in more detail. The substituent definitions are as defined herein.

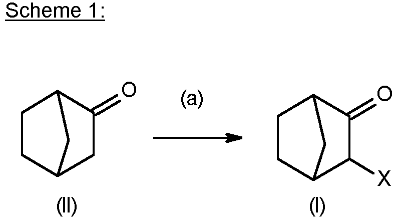

### Step (a) Halogenation:

Compounds of formula (I) can be prepared by reacting a compound of formula (II) 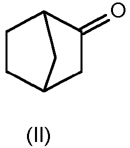 with a halogenating reagent in a suitable reaction medium comprising an organic carbonate to give a compound of formula (I) 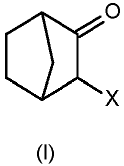 wherein X is as defined herein.

Typically the process described in step (a) is carried out with any halogenation reagent suitable for the preparation of halo-norbornone. Preferably, the process described in step (a) is carried out with a halogenating reagent selected from the group consisting of bromine, chlorine, iodine, N-bromosuccinimide, N-chlorosuccinimide, N-iodosuccinimide, pyridinium bromide tribromide, copper(II) bromide, sulfuryl chloride and trichloroisocyanuric acid. More preferably, the process described in step (a) is carried out with a halogenating reagent selected from the group consisting of bromine, chlorine, N-bromosuccinimide, N-chlorosuccinimide, pyridinium bromide tribromide, copper(II) bromide, sulfuryl chloride and trichloroisocyanuric acid. Even more preferably, the process described in step (a) is carried out with a halogenating reagent selected from the group consisting of bromine, N-bromosuccinimide, pyridinium bromide tribromide and copper(II) bromide. Even more preferably, the process described in step (a) is carried out with a halogenating reagent selected from bromine or pyridinium bromide tribromide. Most preferably, the process described in step (a) is carried out with a halogenating reagent selected from bromine.

Typically the process described in step (a) is carried out in a suitable reaction medium comprising an organic carbonate (or mixture of organic carbonates). Preferably, the process described in step (a) is carried out in a suitable reaction medium comprising an organic carbonate, wherein the organic carbonate is selected from the group consisting of dimethyl carbonate, diethyl carbonate, ethylene carbonate, propylene carbonate, butylene carbonate, diphenyl carbonate and glycerol carbonate (and/or mixtures thereof). More preferably, the process described in step (a) is carried out in a suitable reaction medium comprising an organic carbonate, wherein the organic carbonate is selected from the group consisting of dimethyl carbonate, diethyl carbonate, ethylene carbonate, propylene carbonate, butylene carbonate and glycerol carbonate (and/or mixtures thereof). Even more preferably, the process described in step (a) is carried out in a suitable reaction medium comprising an organic carbonate, wherein the organic carbonate is selected from the group consisting of dimethyl carbonate, diethyl carbonate, ethylene carbonate, propylene carbonate and butylene carbonate (and/or mixtures thereof). Even more preferably still, the process described in step (a) is carried out in a suitable reaction medium comprising an organic carbonate, wherein the organic carbonate is dimethyl carbonate or diethyl carbonate (and/or mixtures thereof). In one embodiment, the process described in step (a) is carried out in a suitable reaction medium comprising an organic carbonate, wherein the organic carbonate is dimethyl carbonate. In another embodiment, the process described in step (a) is carried out in a suitable reaction medium comprising an organic carbonate, wherein the organic carbonate is diethyl carbonate.

Typically this step can be carried out at a temperature of from -20 °C to 150 °C, preferably, from 0 °C to 100 °C, more preferably from 20 °C to 85 °C, even more preferably from 60 °C to 80 °C.

The skilled person would appreciate that compounds of formula (I) may exist as the *exo*-isomer (la) or the *endo*-isomer (Ib) below; 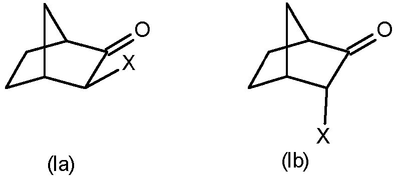 this invention covers processes to prepare all such isomers and mixtures thereof in all proportions.

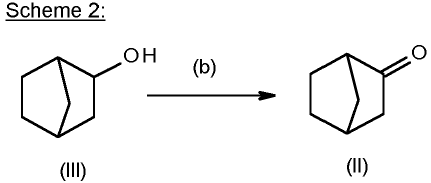

### Step (b) oxidation:

Compounds of formula (II) can be prepared by reaction a compound of formula (III) 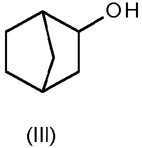 with an oxidising reagent in a suitable reaction medium comprising an organic carbonate to give a compound of formula (II).

Typically the process described in step (b) is carried out with any oxidising reagent suitable for the preparation of norbornone. Suitable oxidising reagents include, but are not limited to hypohalous acid salts such as sodium hypobromite (NaBrO), sodium hypochlorite (NaClO) and potassium hypochlorite (KCIO) and halous acid salts such as sodium bromite (NaBrO₂), sodium chlorite (NaClO₂) and magnesium chlorite (Mg(ClO₂)₂) in the presence of a suitable catalyst.

Preferably, the process described in step (b) is carried out with an oxidising reagent, wherein the oxidising reagent is a hypohalous salt. More preferably, the process described in step (b) is carried out with an oxidising reagent, wherein the oxidising reagent is selected from the group consisting of sodium hypobromite (NaBrO), sodium hypochlorite (NaClO) and potassium hypochlorite (KCIO). Most preferably, the process described in step (b) is carried out with an oxidising reagent selected from sodium hypochlorite (NaClO).

Suitable catalysts that can be used in the process described in step (b) include, but are not limited to a Brönsted acid, such as trifluoroacetic acid, acetic acid, propionic acid, hydrochloric acid and sulfuric acid; nitroxyl radicals such as 2,2,6,6-tetramethylpiperidine-1-oxyl (TEMPO), 4-acetoamido-TEMPO, 4-carboxy-TEMPO, 4-amino-TEMPO, 4-phosphonoxy-TEMPO, 4-(2-bromoacetoamido)-TEMPO, 4-hydroxy-TEMPO, 4-oxy-TEMPO, 3-carboxyl-2,2,5,5-tetramethylpyrrolidin-1-oxyl, 3-carbamoyl-2,2,5,5-tetramethylpyrrolidin-1-oxyl and 3-carbamoyl-2,2,5,5-tetramethyl-3-pyrrolin-1-yloxyl, 1-methyl-2-azaadamantane-N-oxyl, 2-azaadamantan-N-oxyl 9-azabicyclo[3.3.1]nonan-N-oxyl,; and ruthenium-oxide reagents such as Ru/Al₂O₃, tetrapropylammonium perruthenate, tetrapropylammonium perrutehenate/*N*-methylmorpholine-*N*-oxide and RuCl₂(PPh₃)₃/TEMPO.

The amount of catalyst is typically from 0.05 to 50 mol% (based on a compound of formula (III)), preferably from 0.1 to 30 mol%. More preferably, from 0.5 to 15 mol%.

Preferably, the process described in step (b) is carried out in a suitable reaction medium further comprising a Brönsted acid. More preferably, the process described in step (b) is carried out in a suitable reaction medium further comprising an acid selected from the group consisting of acetic acid, hydrochloric acid and sulfuric acid. Most preferably, the process described in step (b) is carried out in a suitable reaction medium further comprising sulfuric acid.

In a preferred embodiment, in the process described in step (b) the oxidising reagent is a hypohalous acid salt and the suitable reaction medium further comprises a Brönsted acid. Preferably, in the process described in step (b) the oxidising reagent is selected from the group consisting of sodium hypobromite (NaBrO), sodium hypochlorite (NaClO) and potassium hypochlorite (KCIO) and the reaction medium further comprises an acid selected from the group consisting of acetic acid, hydrochloric acid and sulfuric acid. Most preferably, in the process described in step (b) the oxidising reagent is sodium hypochlorite (NaClO) and the suitable reaction medium further comprises sulfuric acid.

Typically the process described in step (b) is carried out in a suitable reaction medium comprising an organic carbonate (or mixture of organic carbonates). Preferably, the process described in step (b) is carried out in a suitable reaction medium comprising an organic carbonate, wherein the organic carbonate is selected from the group consisting of dimethyl carbonate, diethyl carbonate, ethylene carbonate, propylene carbonate, butylene carbonate, diphenyl carbonate and glycerol carbonate (and/or mixtures thereof). More preferably, the process described in step (b) is carried out in a suitable reaction medium comprising an organic carbonate, wherein the organic carbonate is selected from the group consisting of dimethyl carbonate, diethyl carbonate, ethylene carbonate, propylene carbonate, butylene carbonate and glycerol carbonate (and/or mixtures thereof). Even more preferably, the process described in step (b) is carried out in a suitable reaction medium comprising an organic carbonate, wherein the organic carbonate is selected from the group consisting of dimethyl carbonate, diethyl carbonate, ethylene carbonate, propylene carbonate and butylene carbonate (and/or mixtures thereof). Even more preferably still, the process described in step (b) is carried out in a suitable reaction medium comprising an organic carbonate, wherein the organic carbonate is dimethyl carbonate or diethyl carbonate (and/or mixtures thereof). In one embodiment, the process described in step (b) is carried out in a suitable reaction medium comprising an organic carbonate, wherein the organic carbonate is dimethyl carbonate. In another embodiment, the process described in step (b) is carried out in a suitable reaction medium comprising an organic carbonate, wherein the organic carbonate is diethyl carbonate.

In a preferred embodiment, the process described in steps (a) and (b) are both carried out in a suitable reaction medium comprising an organic carbonate. Preferably, the process described in steps (a) and (b) are both carried out in a suitable reaction medium comprising an organic carbonate, wherein the organic carbonate is selected from the group consisting of dimethyl carbonate, diethyl carbonate, ethylene carbonate, propylene carbonate, butylene carbonate, diphenyl carbonate and glycerol carbonate (and/or mixtures thereof). More prefarbly, the process described in steps (a) and (b) are both carried out in a suitable reaction medium comprising an organic carbonate, wherein the organic carbonate is selected from the group consisting of dimethyl carbonate, diethyl carbonate, ethylene carbonate, propylene carbonate, butylene carbonate and glycerol carbonate (and/or mixtures thereof). Even more preferably, the process described in steps (a) and (b) are both carried out in a suitable reaction medium comprising an organic carbonate, wherein the organic carbonate is selected from the group consisting of dimethyl carbonate, diethyl carbonate, ethylene carbonate, propylene carbonate and butylene carbonate (and/or mixtures thereof). Yet even more preferably, the process described in steps (a) and (b) are both carried out in a suitable reaction medium comprising an organic carbonate, wherein the organic carbonate is dimethyl carbonate or diethyl carbonate (and/or mixtures thereof). In one embodiment the process described in steps (a) and (b) are both carried out in a suitable reaction medium comprising an organic carbonate, wherein the organic carbonate is dimethyl carbonate. In another embodiment, the process described in steps (a) and (b) are both carried out in a suitable reaction medium comprising an organic carbonate, wherein the organic carbonate is diethyl carbonate.

Typically this step can be carried out at a temperature of from -20 °C to 120 °C, preferably, from -10 °C to 80 °C, more preferably from 0 °C to 50 °C, even more preferably from 10 °C to 30 °C.

The skilled person would appreciate that compounds of formula (III) may exist as the *exo*-isomer (Illa) or the *endo*-isomer (IIIb) below; 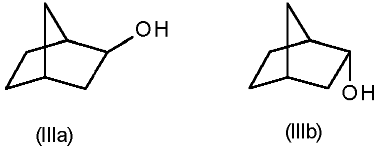 this invention covers processes to oxidise all such isomers and mixtures thereof in all proportions to the corresponding compound (II).

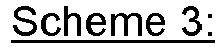

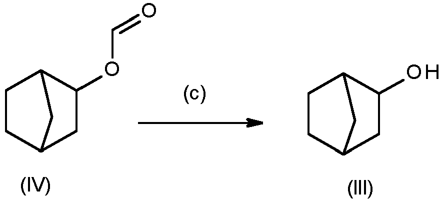

### Step (c) hydrolysis:

Compounds of formula (III) can be prepared by hydrolysis of a compound of formula (IV)

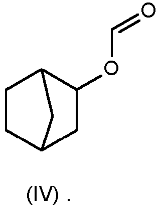

The hydrolysis can be performed using methods known to a person skilled in the art. The hydrolysis is typically performed using suitable conditions, including, but not limited to basic conditions (such as aqueous sodium hydroxide, potassium hydroxide, sodium carbonate or potassium carbonate), or acidic conditions (such as aqueous sulfuric acid). Preferably, the process described in step (c) is carried out in basic conditions. More preferably, the process described in step (c) is carried out with a base selected from the group consisting of sodium hydroxide, potassium hydroxide, sodium carbonate and potassium carbonate. Even more preferably, the the process described in step (c) is carried out with sodium hydroxide.

Typically the process described in step (c) is carried out in the absence of additional solvent (the skilled person would appreciate that the compound of formula (IV) may act as a solvent), or in the presence of a solvent, or mixture of solvents, such as but not limited to, water, acetic acid, propionic acid, methanol, ethanol, propanol, isopropanol, tert-butanol, butanol, 3-methyl-1-butanol, tetrahydrofuran, 2-methyltetrahydrofuran, diethylether, *tert*-butylmethylether, *tert*-amyl methyl ether, cyclopentyl methyl ether, dimethoxymethane, diethoxymethane, dipropoxy methane, 1,3-dioxolane, ethyl acetate, dimethyl carbonate, diethyl carbonate, ethylene carbonate, propylene carbonate, butylene carbonate, diphenyl carbonate, glycerol carbonate, dichloromethane, dichloroethane, *N*,*N*-dimethylformamide, *N,N-*dimethylacetamide, N-methyl pyrrolidone (NMP), acetonitrile, propionitrile, butyronitrile, benzonitrile (or derivative thereof e.g 1,4-dicyanobenzene), 1,4-dioxane or sulfolane. Preferably the process described in step (c) is carried out in the absence of additional solvent, or in the presence of a solvent, or mixture of solvents, selected from the group consisting of water, methanol, ethanol, propanol, isopropanol, tert-butanol, butanol, dimethyl carbonate, diethyl carbonate, acetonitrile, tetrahydrofuran and methyltetrahydrofuran. Preferably the process described in step (c) is carried out in the absence of additional solvent, or in the presence of a solvent, or mixture of solvents, selected from the group consisting of water, methanol, ethanol, propanol, isopropanol, butanol, acetonitrile, tetrahydrofuran and methyltetrahydrofuran.

The skilled person would appreciate that the choice of solvent for the process described in step (c) will depend upon whether basic or acidic coniditons are used.

Typically this step can be carried out at a temperature of from -20 °C to 120 °C, preferably, from -10 °C to 80 °C, more preferably from 0 °C to 50 °C, even more preferably from 10 °C to 30 °C.

The skilled person would appreciate that compounds of formula (IV) may exist as the exo-isomer (IVa) or the endo-isomer (IVb) below; 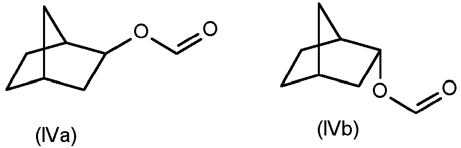 this invention covers processes to hydrolyse all such isomers and mixtures thereof in all proportions to the corresponding compound (III).

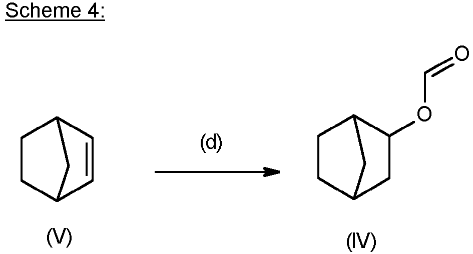

### Step (d) oxy-formylation:

Compounds of formula (IV) can be prepared by oxy-formylation of a compound of formula (V)

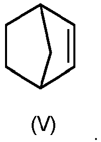

The formylation can be performed using a suitable formylating reagent such as formic acid.

Typically the process described in step (d) is carried out in the absence of additional solvent (the skilled person would appreciate that the compound of formula (V) or the formylating reagent may act as a solvent), or in the presence of a solvent, or mixture of solvents, such as but not limited to, water, acetic acid, propionic acid, methanol, ethanol, propanol, isopropanol, butanol, 3-methyl-1-butanol, tetrahydrofuran, 2-methyltetrahydrofuran, diethylether, cyclopentyl methyl ether, ethyl acetate, dimethyl carbonate, diethyl carbonate, ethylene carbonate, propylene carbonate, butylene carbonate, diphenyl carbonate, glycerol carbonate, dichloromethane, dichloroethane, *N*,*N*-dimethylformamide, *N,N-*dimethylacetamide, N-methyl pyrrolidone (NMP), acetonitrile, propionitrile, butyronitrile, benzonitrile (or derivative thereof e.g 1,4-dicyanobenzene), 1,4-dioxane or sulfolane. Preferably the process described in step (d) is carried out in the absence of additional solvent, or in the presence of a solvent, or mixture of solvents, selected from the group consisting of water, methanol, ethanol, propanol, isopropanol, butanol, dimethyl carbonate, diethyl carbonate, acetonitrile, tetrahydrofuran and methyltetrahydrofuran.

Typically this step can be carried out at a temperature of from 50 °C to 120 °C, preferably, from 70 °C to 100 °C.

The skilled person would appreciate that compounds of formula (IV) may exist as the *exo*-isomer (IVa) or the *endo*-isomer (IVb). This invention covers processes to prepare all such isomers and mixtures thereof in all proportions from the compound (V).

In a preferred embodiment of the process described in step (d) excess formylating reagent (preferably formic acid) is distilled off upon completion of the reaction.

The skilled person would appreciate that the temperature of the process according to the invention can vary in each of steps (a), (b), (c) and (d). Furthermore, this variability in temperature may also reflect the choice of solvent used. Likewise, the skilled person would also appreciate that the pressure of the process according to the invention can vary in each of steps (a), (b), (c) and (d) depending on the choice of solvent and temperature used. Typically, the process according to the invention is conducted at a pressure from 1 to 20 bar.

Preferably, the process of the present invention is carried out under an inert atmosphere, such as nitrogen or argon.

The skilled person would appreciate that process steps (a), (b) and (c) can be carried out in separate process steps, wherein the intermediate compounds can be isolated at each stage. Alternatively, the process steps (a), (b) and (c) can be carried out in a telescoped procedure wherein the intermediate compounds produced are not isolated. Thus, it is possible for the process of the present invention to be conducted in a batch wise, semi-batch wise or continuous fashion.

The skilled person would appreciate that steps (a), (b), (c) and (d) could equally be represented in a single scheme, see scheme 5 or scheme 6 below,

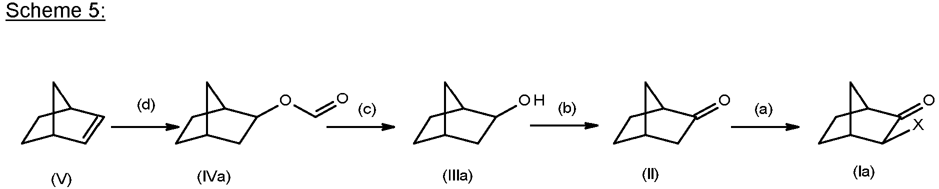

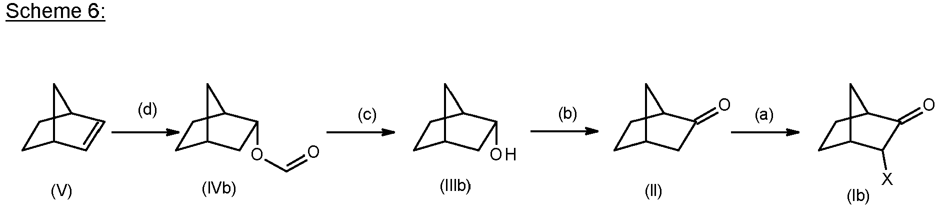

In a preferred embodiment of the invention there is provided a process for the preparation of a compound of formula (I), 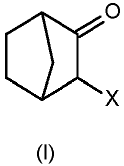 wherein X is bromine;
said process comprising:
reacting a compound of formula (II), 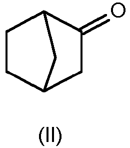
with a halogenating reagent selected from the group consisting of bromine, N-bromosuccinimide, pyridinium bromide tribromide and copper(II) bromide (preferably, bromine or pyridinium bromide tribromide, most preferably bromine) in a suitable reaction medium comprising an organic carbonate wherein the organic carbonate is dimethyl carbonate or diethyl carbonate (preferably, dimethyl carbonate), to give a compound of formula (I).

In a preferred embodiment of the invention there is provided a process for the preparation of a compound of formula (I), 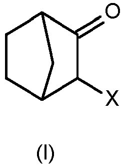 wherein X is bromine;
said process comprising the steps of:
(i) reacting a compound of formula (III) 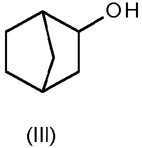 with an oxidising reagent, wherein the oxidising reagent is a hypohalous acid salt (preferably, sodium hypobromite (NaBrO), sodium hypochlorite (NaClO) or potassium hypochlorite (KCIO), more preferably sodium hypochlorite (NaClO)) in a suitable reaction medium comprising an organic carbonate, wherein the organic carbonate is dimethyl carbonate or diethyl carbonate (preferably, dimethyl carbonate), and wherein the suitable reaction medium further comprises a Brönsted acid (preferably, acetic acid, hydrochloric acid or sulfuric acid, more preferably sulfuric acid) to give a compound of formula (II), 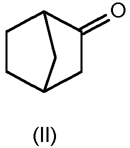 and
(ii) reacting a compound of formula (II), with a halogenating reagent selected from the group consisting of bromine, N-bromosuccinimide, pyridinium bromide tribromide and copper(II) bromide (preferably, bromine or pyridinium bromide tribromide, most preferably bromine) in a suitable reaction medium comprising an organic carbonate wherein the organic carbonate is dimethyl carbonate or diethyl carbonate (preferably, dimethyl carbonate), to give a compound of formula (I).

In a further preferred embodiment of the invention there is provided a process for the preparation of a compound of formula (I), 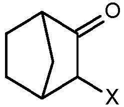 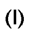 wherein X is bromine;
said process comprising the steps of:
(i) hydrolysis of a compound of formula (IV) 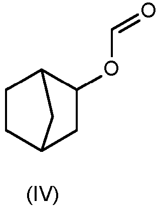 to give a compound of formula (III), 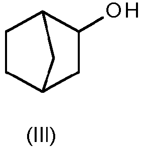 under basic conditions (preferably, with aqueous sodium hydroxide, potassium hydroxide, sodium carbonate or potassium carbonate, more preferably with aqueous sodium hydroxide);
(ii) reacting a compound of formula (III), with an oxidising reagent, wherein the oxidising reagent is a hypohalous acid salt (preferably, sodium hypobromite (NaBrO), sodium hypochlorite (NaClO) or potassium hypochlorite (KCIO), more preferably sodium hypochlorite (NaClO)) in a suitable reaction medium comprising an organic carbonate, wherein the organic carbonate is dimethyl carbonate or diethyl carbonate (preferably, dimethyl carbonate), and wherein the suitable reaction medium further comprises a Brönsted acid (preferably, acetic acid, hydrochloric acid or sulfuric acid, more preferably sulfuric acid) to give a compound of formula (II), 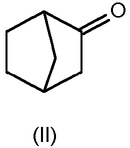 and
(iii) reacting a compound of formula (II), with a halogenating reagent selected from the group consisting of bromine, N-bromosuccinimide, pyridinium bromide tribromide and copper(II) bromide (preferably, bromine or pyridinium bromide tribromide, most preferably bromine) in a suitable reaction medium comprising an organic carbonate wherein the organic carbonate is dimethyl carbonate or diethyl carbonate (preferably, dimethyl carbonate), to give a compound of formula (I).

In a further preferred embodiment of the invention there is provided a process for the preparation of a compound of formula (I), 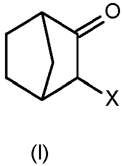 wherein X is bromine;
said process comprising the steps of:
(i) reacting a compound of formula (V) 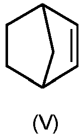 with formic acid to give a compound of formula (IV) 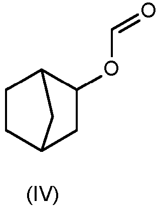
(ii) hydrolysis of a compound of formula (IV) to give a compound of formula (III), 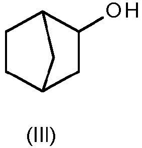 under basic conditions (preferably, with sodium hydroxide, potassium hydroxide, sodium carbonate or potassium carbonate, more preferably with sodium hydroxide);
(iii) reacting a compound of formula (III), with an oxidising reagent, wherein the oxidising reagent is a hypohalous acid salt (preferably, sodium hypobromite (NaBrO), sodium hypochlorite (NaClO) or potassium hypochlorite (KCIO), more preferably sodium hypochlorite (NaClO)) in a suitable reaction medium comprising an organic carbonate, wherein the organic carbonate is dimethyl carbonate or diethyl carbonate (preferably, dimethyl carbonate), and the suitable reaction medium further comprises a Brönsted acid (preferably, acetic acid, hydrochloric acid or sulfuric acid, more preferably sulfuric acid) to give a compound of formula (II), 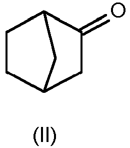 and
(iv) reacting a compound of formula (II), with a halogenating reagent selected from the group consisting of bromine, N-bromosuccinimide, pyridinium bromide tribromide and copper(II) bromide (preferably, bromine or pyridinium bromide tribromide, most preferably bromine) in a suitable reaction medium comprising an organic carbonate wherein the organic carbonate is dimethyl carbonate or diethyl carbonate (preferably, dimethyl carbonate), to give a compound of formula (I).

In a further embodiment of the invention the process further comprises converting a compound of formula (I) to a compound of formula (VI), 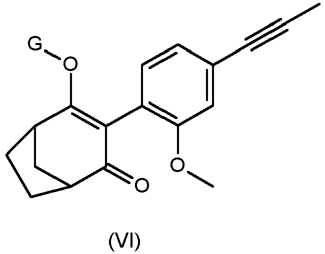
wherein G is selected from the group consisting of hydrogen, C₂-C₆alkenyl, C₂-C₆alkynyl, C₁-C₃alkoxyC₁-C₃alkyl-, -C(O)-R¹, -C(O)-X^{a}-R¹ and -S(O)₂-R¹;
X^{a} is oxygen or sulfur; and
R¹ is selected from the group consisting of C₁-C₆alkyl, C₂-C₆alkenyl, phenyl and 4-fluorophenyl.

In a preferred embodiment of the invention, the process further comprises converting a compound of formula (I) to a compound of formula (VI), 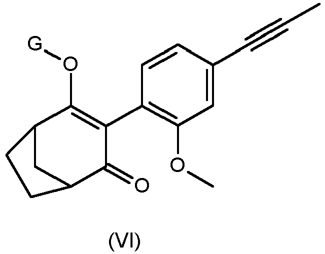
wherein G is selected from the group consisting of hydrogen, C₂-C₆alkenyl, C₂-C₆alkynyl, C₁-C₃alkoxyC₁-C₃alkyl-, -C(O)-R¹, -C(O)-X^{a}-R¹ and -S(O)₂-R¹;
X^{a} is oxygen or sulfur; and
R¹ is selected from the group consisting of C₁-C₆alkyl, C₂-C₆alkenyl, phenyl and 4-fluorophenyl;
wherein the process further comprises the steps as described on page 54 of WO 2015/197468.

Preferably, the process further comprises converting a compound of formula (I) to a compound of formula (VI), 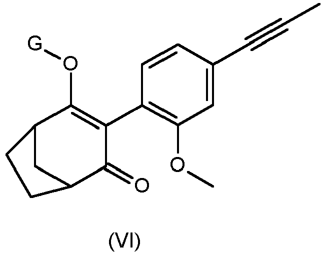
wherein G is hydrogen or -C(O)-R¹ (preferably, G is -C(O)-R¹);
X^{a} is oxygen; and
R¹ is selected from the group consisting of C₁-C₆alkyl and C₂-C₆alkenyl (preferably, R¹ is C₁-C₆alkyl);
wherein the process further comprises the steps as described on page 54 of WO 2015/197468.

### Examples:

The following examples further illustrate, but do not limit the invention. Those skilled in the art will promptly recognise appropriate variations from the procedures both as to the reactants and as to the reaction conditions and techniques.

The following abbreviations are used: s = singlet; br s = broad singlet; d = doublet; dd = double doublet; dt = double triplet; t = triplet, tt = triple triplet, q = quartet, quin = quintuplet, sept = septet; m = multiplet; GC = gas chromatography, RT = retention time, Tᵢ = internal temperature, MH⁺ = molecular mass of the molecular cation, M = molar, Q¹HNMR = quantitative ¹H Nuclear Magnetic Resonance, RT = room temperature, UFLC = Ultra-fast liquid chromatography.

¹H NMR spectra are recorded at 400 MHz unless indicated otherwise and chemical shifts are recorded in ppm.

Some chemical yields have been calculated precisely using quantitative ¹H NMR and 1,3,5-trimethoxybenzene or caffeine as an internal standard.

Conversion was followed by gas chromatography with analysis being conducted on Agilent GC Model 6850 using Agilent 19091U-211 type column with DB-1701 description. Equipment detector FID operated with hydrogen at 300 °C. Method hold 2 min at 40 °C, 20°C/min until 300°C, hold 2 min at 300°C, total time 19min.

### Example 1 - Preparation of norbornan-2-yl formate from norbornene

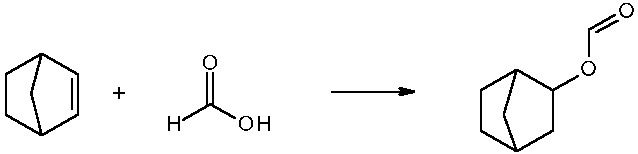

To a 1.5 L double mantled reactor equipped with an overhead stirrer, reflux condenser and heated addition funnel, was added technical formic acid (88 wt%) (577 g, 11.0 mol, 3.5 equiv) and Tᵢₙₜ was set to 90 °C. Once the temperature has been reached, molten norbornene (300 g, 3.1 mol, 1.0 equiv) was added dropwise over the course of 2h. Upon addition the reaction was allowed to stir at this temperature for additional 2 h and the excess formic acid was distilled off under reduced pressure, to yield (386 g, 95% purity) product as a colorless-pale yellow oil, accounting for 83% isolated yield. Product contained 3% of norbornan-2-ol that is target product in subsequent step.

Distillate contained ~10 wt% of product that together with the recovered formic acid can be reused in a subsequent batch. Furthermore, the use of distillation column can diminish the amount of product in the distillate.

Product purity was determined using quantitative ¹H NMR using 1,3,5-trimethoxybenzene as an internal standard.

¹H, NMR (400 MHz, CDCl₃) δ ppm: 7.98 (s, 1H), 4.71 (m, 1H), 2.33 (m, 2H), 1.80 - 1.70 (m, 1H), 1.60 - 1.40 (m, 4H), 1.20 - 0.95 (m, 3H).

### Example 2 - Preparation of norbornan-2-ol from norbornan-2-yl formate

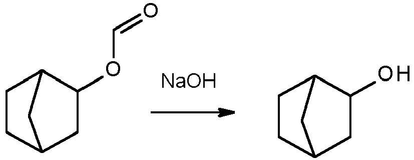

To a 1L double mantled reactor equipped with an overhead stirrer, reflux condenser, internal pH probe and addition funnel was added crude norbornan-2-yl formate (110 g, 0.8 mol, 1.0 equiv). To this mixture was added 10% aq NaOH (324 g, 0.88 mol, 1.1 equiv) at Tᵢₙₜ = 25 °C over the course of 2h. Cooling is required to maintain this temperature however can be omitted as reaction proceeds at higher temperature equally well. More water can be added if reaction mass becomes difficult to stir. After full addition the reaction mixture is additionally stirred at 25 °C for 1 h. Then 10% aq HCl (15.0 g, 0.04 mol, 0.05 equiv) is added dropwise to set the pH = 8. Once pH is reached, dimethylcarbonate 264 g is added in 1 portion. Phases are separated and organic layer is washed with 1 x 50 g water.

Product is obtained as a ~20wt% solution in DMC (359 g, 86% isolated yield).

Small sample is concentrated under reduced pressure for analytical purposes - Product purity was determined to be 92 % using quantitative ¹H NMR with 1,3,5-trimethoxybenzene as an internal standard.

¹H, NMR (400 MHz, CDCl₃) δ ppm: 3.75 (m, 1H), 2.25 (m, 1H), 2.12 (m, 1H), 1.66 (m, 2H), 1.56 (m, 1H), 1.50 - 1.35 (m, 2H), 1.29 (m, 1H), 1.12 (m, 1H), 1.95 - 0.95 (m, 2H).

*Note: reaction can be carried out by dosing norbornan-2-yl formate to 10% aq NaOH.*

### Exampe 3 - Preparation of norbornan-2-one from norbornan-2-ol

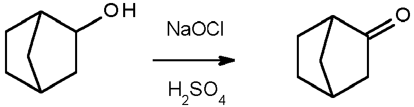

To a 1.5 L double mantled reactor equipped with an overhead stirrer, reflux condenser and addition funnel was added 23wt% (in DMC) solution of norbornol (350 g, 0.73 mol, 1.0 equiv) followed by 10% aq sulfuric acid (139g, 0.14 mol, 0.2 equiv). To this mixture is added ~10wt% aq NaOCl (553 g, 0.77mol, 1.05 equiv) at Tᵢₙₜ = 25 °C over the course of 2h. Cooling is required to maintain the reaction temperature. Upon completion reaction is additionally stirred at 25 °C for 1 h. Then phases are separated and organic layer is directly used in the following step.

Product content was determined to be 26 wt % (solution in DMC) using quantitative ¹H NMR with 1,3,5-trimethoxybenzene as an internal standard. This product was directly subjected to the following bromination.

Typical product purity for the product of this procedure was determined to be in the range of from 90 % to 95 % using quantitative ¹H NMR with 1,3,5-trimethoxybenzene as an internal standard.

¹H, NMR (400 MHz, CDCl₃) δ ppm: 2.65 (m, 1H), 2.59 (m, 1H), 2.05 (m, 1H), 1.85 - 1.70 (m, 4H), 1.58 - 1.38 (m, 3H).

If required a higher concentration aq NaOCl solution can be used to improve the process volume yield.

### Example 4 - Preparation of 3-bromonorbornan-2-one from norbornan-2-one

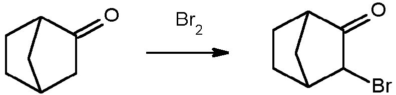

To a 0.5 L double mantled reactor equipped with an overhead stirrer, reflux condenser, off-gas scrubber and addition funnel was added 26wt% (in DMC) solution of norbornan-2-one (150 g, 0.35 mol, 1.00 equiv) and Tᵢₙₜ is set to 75 °C. Then neat bromine (57g, 0.35 mol, 1.00 equiv) is added dropwise over the course of 2h. Gas evolution is observable with every drop of bromine added. Conversion is measured by GC. To assure full conversion of the unreacted starting material additional bromine (1.6 g, 0.01 mol, 0.03 equiv) was added. Upon complete addition of bromine the reaction is additionally stirred for 1h at 75 °C before it is cooled to 25 °C and argon is bubbled through to eliminate residual HBr. Solution is washed 1 x 20 g 10% aq NaHSO₃, phases are separated and organic solvent is removed under reduced pressure to afford product as a brown liquid (68 g, 89% isolated yield). Product purity was determined to be 88% using quantitative ¹H NMR with 1,3,5-trimethoxybenzene as an internal standard.

The crude product is then additionally purified by overhead distillation at Tᵢₙₜ = 80 °C and 2mbar pressure to afford target material as a colourless oil.

¹H, NMR (400 MHz, CDCl₃) δ ppm: 3.83 (d, *J* = 3.2 Hz, 1H), 2.79 (m, 1H), 2.72 (m, 1H), 2.29 (m, 1H), 1.98 (m, 1H), 1.80 (m, 1H), 1.65 - 1.40 (m, 3H).

¹H NMR coupling constant analysis and estimations of dihedral angles indicates that product is obtained as predominantly exo- isomer (compared to the endo-isomer, a compound of formula (Ib-I)) with bromine atom adapting equatorial position, a compound of formula (Ia-I) below,

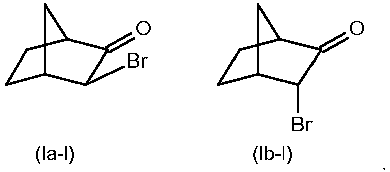

General reaction conditions for the solvent comparison described in Table 1 below are based on the process described in Example 4.

All experiments were carried out on a scale of from 2.5 to 10.0 g of norbornane-2-one.

A solution of norbornan-2-one (1.00 equiv) in solvent (as listed in table below) is heated to Ti = 75 - 80 °C at which point bromine (1.05 - 1.10 equiv) is added over a course of 1-2h. The reaction is allowed to stir for an additional hour and then is sampled for GC analysis.

**Table 1 - Solvent effect on selectivity of bromination**

| **Solvent** | **SM (%)** | **Prod (%)** | **Side Prod (%)** | **Selectivity (%)** | **Reactivity (%)** | **S/R factor** |
|---|---|---|---|---|---|---|
| Acetic Acid | 0 | 78.4 | 21.6 | 78 | 100 | 3.6 |
| Methylcyclohexane | 14.6 | 79.4 | 6.0 | 93 | 84 | 3.9 |
| Heptane* | 88 | 12 | - | - | - | - |
| Cyclohexane | 11.3 | 81.7 | 7.0 | 92 | 88 | 4.5 |
| Dimethylcarbonate | 0.2 | 93.8 | 5.9 | 94 | 100 | 15.2 |
| Diethylcarbonate | 1.8 | 93.2 | 5.0 | 95 | 98 | 13.8 |

| | | | | | | |
|---|---|---|---|---|---|---|
| *Bromine consumed by the reaction solvent. | | | | | | |

Analysis was done by Gas Chromatography and in all cases only 3 signals analyzed - starting material, norbornan-2-one (SM), 3-bromonorbornan-2-one (Prod) and over brominated side product, 3,3-dibromonorbornan-2-one, a compound of formula (VII) (side prod).

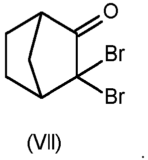

While not included in Table 1, the reactions in alkane solvents (methycyclohexane, heptane and cyclohexane) exhibit more impurities including bromination of the reaction solvent.
In Table 1 Reactivity is expressed as %Prod/(%Prod+%Starting material) * 100
In Table 1 Selectivity is expressed as %Prod/(%Prod + % Side products) * 100
In Table 1 Selecitivity is linked with Reactivity in a comparative S/R (Selectivity/Reactivity) parameter that is expressed as %Prod/(%Starting material + %Side product)

The above results demonstrate that the organic carbonate solvents used in the process of the invention achieve high selectivity (reduced side products) and conversion to the desired product.

## Claims

1. A process for the preparation of a compound of formula (I), 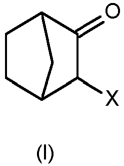 wherein X is halogen;
said process comprising:
reacting a compound of formula (II), 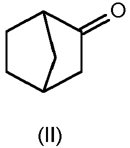
with a halogenating reagent in a suitable reaction medium comprising an organic carbonate, to give a compound of formula (I).

2. A process according to claim 1, wherein X is chlorine or bromine.

3. A process according to claim 1 or claim 2, wherein X is bromine.

4. A process according to claim 1, wherein the halogenating reagent is selected from the group consisting of bromine, chlorine, iodine, N-bromosuccinimide, N-chlorosuccinimide, N-iodosuccinimide, pyridinium bromide tribromide, copper(II) bromide, sulfuryl chloride and trichloroisocyanuric acid.

5. A process according to claim 3, wherein the halogenating reagent is selected from the group consisting of bromine, N-bromosuccinimide, pyridinium bromide tribromide and copper(II) bromide.

6. A process according to claim 3 or claim 5, wherein the halogenating reagent is bromine.

7. A process according to any one of claims 1 to 6, wherein the compound of formula (II) is prepared by reaction of a compound of formula (III), 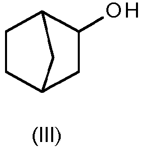 with an oxidising reagent in a suitable reaction medium comprising an organic carbonate.

8. A process according to claim 7 wherein the oxidising reagent is sodium hypochlorite.

9. A process according to claim 8, wherein the reaction medium further comprises sulfuric acid.

10. A process according to any one of claims 1 to 9, wherein the organic carbonate is selected from the group consisting of dimethyl carbonate, diethyl carbonate, ethylene carbonate, propylene carbonate, butylene carbonate and glycerol carbonate.

11. A process according to any one of claims 1 to 10, wherein the organic carbonate is selected from the group consisting of dimethyl carbonate and diethyl carbonate.

12. A process according to any one of claims 1 to 11, wherein the intermediate compounds of formula (II) and/or (III) are not isolated.

13. A process according to any one of claims 7 to 12, wherein the compound of formula (III) is prepared by:
(i) reacting a compound of formula (IV), 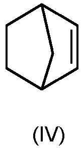 with formic acid to give a compound of formula (V), 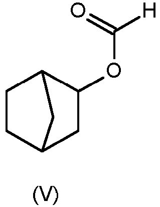 and
(ii) hydrolysing the compound of formula (V) to a compound of formula (III).

14. A process according to claim 13, wherein the hydrolysis step is performed with sodium hydroxide.

15. A process according to any one of claims 1 to 14, wherein the process further comprises converting a compound of formula (I) to a compound of formula (VI), 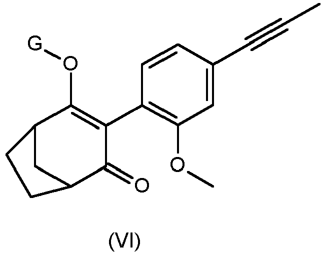
Wherein G is selected from the group consisting of hydrogen, C₂-C₆alkenyl, C₂-C₆alkynyl, C₁-C₃alkoxyC₁-C₃alkyl-, -C(O)-R¹, -C(O)-X^{a}-R¹ and -S(O)₂-R¹;
X^{a} is oxygen or sulfur; and
R¹ is selected from the group consisting of C₁-C₆alkyl, C₂-C₆alkenyl, phenyl and 4-fluorophenyl.

## Patentansprüche

1. Verfahren zur Herstellung einer Verbindung der Formel (I), 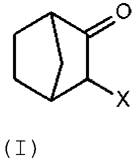
wobei X für Halogen steht;
wobei das Verfahren Folgendes umfasst:
Umsetzen einer Verbindung der Formel (II) 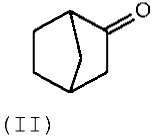
mit einem Halogenierungsmittel in einem geeigneten Reaktionsmedium, das ein organisches Carbonat umfasst, so dass eine Verbindung der Formel (I) erhalten wird.

2. Verfahren nach Anspruch 1, wobei X für Chlor oder Brom steht.

3. Verfahren nach Anspruch 1 oder Anspruch 2, wobei X für Brom steht.

4. Verfahren nach Anspruch 1, wobei das Halogenierungsmittel aus der Gruppe bestehend aus Brom, Chlor, Iod, N-Bromsuccinimid, N-Chlorsuccinimid, N-Iodsuccinimid, Pyridiniumbromidtribromid, Kupfer(II)bromid, Sulfurylchlorid und Trichlorisocyanursäure ausgewählt ist.

5. Verfahren nach Anspruch 3, wobei das Halogenierungsmittel aus der Gruppe bestehend aus Brom, N-Bromsuccinimid, Pyridiniumbromidtribromid und Kupfer(II)bromid ausgewählt ist.

6. Verfahren nach Anspruch 3 oder Anspruch 5, wobei es sich bei dem Halogenierungsmittel um Brom handelt.

7. Verfahren nach einem der Ansprüche 1 bis 6, wobei die Verbindung der Formel (II) durch Umsetzung einer Verbindung der Formel (III) 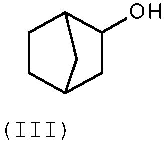 mit einem Oxidationsmittel in einem geeigneten Reaktionsmedium, das ein organisches Carbonat umfasst, hergestellt wird.

8. Verfahren nach Anspruch 7, bei dem es sich bei dem Oxidationsmittel um Natriumhypochlorit handelt.

9. Verfahren nach Anspruch 8, wobei das Reaktionsmedium ferner Schwefelsäure umfasst.

10. Verfahren nach einem der Ansprüche 1 bis 9, wobei das organische Carbonat aus der Gruppe bestehend aus Dimethylcarbonat, Diethylcarbonat, Ethylencarbonat, Propylencarbonat, Butylencarbonat und Glycerincarbonat ausgewählt ist.

11. Verfahren nach einem der Ansprüche 1 bis 10, wobei das organische Carbonat aus der Gruppe bestehend aus Dimethylcarbonat und Diethylcarbonat ausgewählt ist.

12. Verfahren nach einem der Ansprüche 1 bis 11, wobei die Zwischenverbindungen der Formel (II) und/oder (III) nicht isoliert werden.

13. Verfahren nach einem der Ansprüche 7 bis 12, wobei die Verbindung der Formel (III) hergestellt wird durch:
(i) Umsetzen einer Verbindung der Formel (IV) 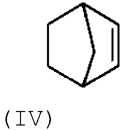 mit Ameisensäure, so dass eine Verbindung der Formel (V) erhalten wird, 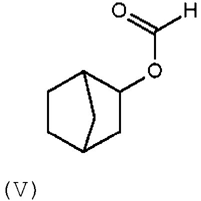 und
(ii) Hydrolysieren der Verbindung der Formel (V) zu einer Verbindung der Formel (III).

14. Verfahren nach Anspruch 13, wobei der Hydrolyseschritt mit Natriumhydroxid durchgeführt wird.

15. Verfahren nach einem der Ansprüche 1 bis 14, wobei das Verfahren ferner Überführen einer Verbindung der Formel (I) in eine Verbindung der Formel (VI) umfasst, 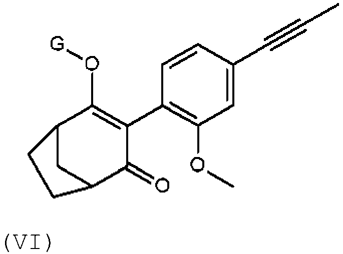
wobei G aus der Gruppe bestehend aus Wasserstoff, C₂-C₆Alkenyl, C₂-C₆Alkinyl, C₁-C₃Alkox_{Y}C₁-C₃alkyl-, -C(O)-R¹, -C(O)-X^{a}-R¹ und -S(O)₂-R¹ ausgewählt ist,
X^{a} für Sauerstoff oder Schwefel steht und
R¹ aus der Gruppe bestehend aus C₁-C₆Alkyl, C₂-C₆Alkenyl, Phenyl and 4-Fluorphenyl ausgewählt ist.

## Revendications

1. Procédé de préparation d'un composé de formule (I) 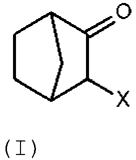
dans laquelle X est halogène ;
ledit procédé comprenant :
la mise en réaction d'un composé de formule (II), 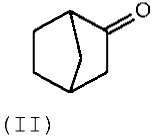
avec un réactif d'halogénation dans un milieu réactionnel approprié comprenant un carbonate organique, pour donner un composé de formule (I).

2. Procédé selon la revendication 1, dans lequel X est chlore ou brome.

3. Procédé selon la revendication 1 ou la revendication 2, dans lequel X est brome.

4. Procédé selon la revendication 1, dans lequel le réactif d'halogénation est choisi dans le groupe constitué par brome, chlore, iode, N-bromosuccinimide, N-chlorosuccinimide, N-iodosuccinimide, tribromure de bromure de pyridinium, bromure de cuivre(II), chlorure de sulfuryle et acide trichloroisocyanurique.

5. Procédé selon la revendication 3, dans lequel le réactif d'halogénation est choisi dans le groupe constitué par brome, N-bromosuccinimide, tribromure de bromure de pyridinium et bromure de cuivre(II).

6. Procédé selon la revendication 3 ou la revendication 5, dans lequel le réactif d'halogénation est le brome.

7. Procédé selon l'une quelconque des revendications 1 à 6, dans lequel le composé de formule (II) est préparé par réaction d'un composé de formule (III), 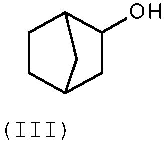 avec un réactif d'oxydation dans un milieu réactionnel approprié comprenant un carbonate organique.

8. Procédé selon la revendication 7, dans lequel le réactif d'oxydation est l'hypochlorite de sodium.

9. Procédé selon la revendication 8, dans lequel le milieu réactionnel comprend en outre de l'acide sulfurique.

10. Procédé selon l'une quelconque des revendications 1 à 9, dans lequel le carbonate organique est choisi dans le groupe constitué par le carbonate de diméthyle, le carbonate de diéthyle, le carbonate d'éthylène, le carbonate de propylène, le carbonate de butylène et le carbonate de glycérol.

11. Procédé selon l'une quelconque des revendications 1 à 10, dans lequel le carbonate organique est choisi dans le groupe constitué par le carbonate de diméthyle et le carbonate de diéthyle.

12. Procédé selon l'une quelconque des revendications 1 à 11, dans lequel les composés intermédiaires de formule (II) et/ou (III) ne sont pas isolés.

13. Procédé selon l'une quelconque des revendications 7 à 12, dans lequel le composé de formule (III) est préparé par :
(i) mise en réaction d'un composé de formule (IV), 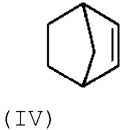 avec de l'acide formique pour donner un composé de formule (V), 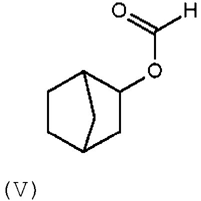 et
(ii) hydrolyse du composé de formule (V) en un composé de formule (III).

14. Procédé selon la revendication 13, dans lequel l'étape d'hydrolyse est effectuée avec de l'hydroxyde de sodium.

15. Procédé selon l'une quelconque des revendications 1 à 14, dans lequel le procédé comprend en outre la conversion d'un composé de formule (I) en un composé de formule (VI), 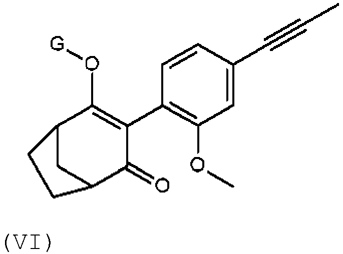
dans laquelle G est choisi dans le groupe constitué par hydrogène, C₂-C₆alcényle, C₂-C₆alcynyle, C₁-C₃alcoxyC₁-C₃alkyle-, -C(O)-R¹, -C(O)-X^{a}-R¹ et -S(O)₂-R¹ ;
X^{a} est oxygène ou soufre ; et
R¹ est choisi dans le groupe constitué par C₁-C₆alkyle, C₂-C₆alcényle, phényle et 4-fluorophényle.
